# EUROPEAN PATENT APPLICATION

(11) **EP 4 296 919 A1**
(43) Date of publication of application: **27.12.2023**
(21) Application number: 23162884.3
(22) Date of filing: 20.03.2023
(51) Int. Cl.: G06Q 10/0631, G06Q 10/0633

(54) **WORKFLOW DISPLAY METHOD, WORKFLOW DISPLAY PROGRAM, AND CORRESPONDING INFORMATION PROCESSING APPARATUS**

(30) Priority: 23.06.2022 JP 2022100887
(71) Applicant: FUJITSU LIMITED, Kawasaki-shi, Kanagawa 211-8588 (JP)
(72) Inventor: AMEMIYA, Satoshi, Kawasaki-shi, Kanagawa, 211-8588 (JP); MIZOUCHI, Tsuyoshi, Kawasaki-shi, Kanagawa, 211-8588 (JP); TADA, Atsuko, Kawasaki-shi, Kanagawa, 211-8588 (JP); INOMATA, Akihiro, Kawasaki-shi, Kanagawa, 211-8588 (JP)
(74) Representative: Hoffmann Eitle

(57) **Abstract**

A display method includes: accepting designation of a first workflow; first counting, by applying a plurality of objects to the first workflow, the number of object distributed by an element of a conditional branch of the first workflow for each of element at an end out of elements included in the first workflow; second counting, by applying the plurality of objects to a second workflow that is different from the first workflow, the number of objects distributed by an element of a conditional branch of the second workflow for each of element at an end out of elements included in the second workflow; and displaying the number of objects distributed to an element at the end, associating with the element at the end, for each of the first workflow and the second workflow.

## Description

### [Technical Field]

The embodiments discussed herein are related to a display method and a display program.

### [Background Art]

A technique of supporting to plan a measure flow in various fields, such as medicine, nursing, and administration, as a workflow has been available. For example, for selection of an energy saving measure, an energy-saving-evaluation supporting device to search for measures that can be introduced to a building from input information of a target building and facilities is proposed (for example, Japanese Laid-open Patent Publication No. 2012-27536) .

### [Summary of invention]

### [Technical Problem]

However, because conventional techniques represented by the energy-saving-evaluation supporting device described above and the like are to only search for related measures that can be related to a measure to be planned, there is an aspect that it is not necessarily possible to provide information contributing to judgement on excess and deficiency of resources.

For example, a measure flow has an aspect that persons subject to measures are distributed to a service or the like to achieve a goal of the measures through conditional branches defined in the measure flow. However, the related measures described above only resemble in scale and kind of facilities of a building to the measure to be planned. Therefore, it is unclear whether the measure flow of the related measure described above is appropriate as an index to judge whether resources, such as service providers, can be distributed appropriately to the number of people to be distributed to a service of the measure flow to be planned. Even if a measure flow of such a related measure is presented, it is difficult to judge excess and deficiency of resources.

A measure flow is used as an example of workflow and person is used as an example of an object to be distributed by the workflow herein, but also in a case in which an object other than person is distributed in an entire workflow, a similar problem can occur.

Accordingly, it is an object in one aspect of an embodiment of the present invention to provide a display method and a display program that can provide information contributing to judgment of excess and deficiency of resources.

### [Solution to Problem]

An aspect of a display method includes: accepting designation of a first workflow; first counting, by applying a plurality of objects to the first workflow, the number of object distributed by an element of a conditional branch of the first workflow for each of element at an end out of elements included in the first workflow; second counting, by applying the plurality of objects to a second workflow that is different from the first workflow, the number of objects distributed by an element of a conditional branch of the second workflow for each of element at an end out of elements included in the second workflow; and displaying the number of objects distributed to an element at the end, associating with the element at the end, for each of the first workflow and the second workflow.

### [Advantageous Effects of Invention]

Information contributing to judgment of excess and deficiency of resources can be provided.

### [Brief Description of Drawings]

FIG. 1 is a block diagram illustrating a functional configuration example of a server device;
FIG. 2 is a diagram illustrating a display example of an original flow and a comparative flow;
FIG. 3 is a diagram illustrating an example of an original flow;
FIG. 4 is a diagram illustrating an example of resident data;
FIG. 5 is a schematic diagram illustrating an example of route identification;
FIG. 6 is a schematic diagram illustrating a calculation example of a degree of difference;
FIG. 7 is a schematic diagram illustrating a calculation example of a degree of difference;
FIG. 8 is a diagram illustrating an example of a comparative flow;
FIG. 9 is a diagram illustrating an example of a relationship between route and frequency;
FIG. 10 is a diagram illustrating an example of a comparative flow;
FIG. 11 is a diagram illustrating an example of a relationship between route and frequency;
FIG. 12 is a diagram illustrating an example of an original flow and a comparative flow;
FIG. 13 is a diagram illustrating a display example of an original flow;
FIG. 14 is a diagram illustrating a display example of a comparative flow;
FIG. 15 is a flowchart illustrating a procedure of display processing; and
FIG. 16 is a diagram illustrating a hardware configuration example.

### [Embodiments for Carrying Out the Invention]

Preferred embodiments will be explained with reference to accompanying drawings. The respective embodiments only illustrate one example and aspect, and numeric values, the scope of functions, and usage scenes are not limited to these exemplifications. The respective embodiments can be combined within a range not causing contradiction in processing.

### First Embodiment

### System Configuration

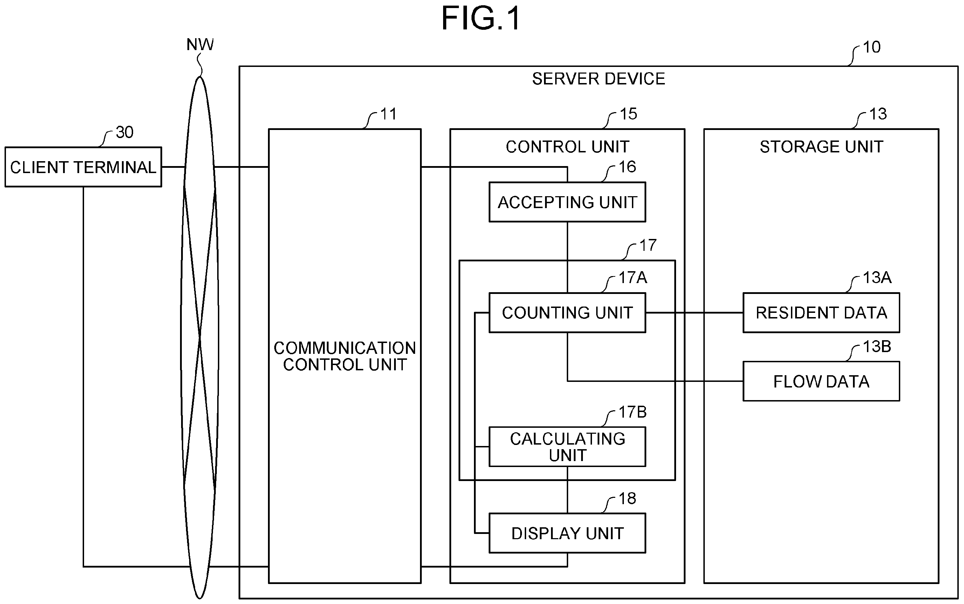

FIG. 1 is a block diagram illustrating a functional configuration example of a server device 10. The server device 10 illustrated in FIG. 1 provides a creation supporting function to support creation of a workflow.

The server device 10 is one example of a computer that provides the creation supporting function described above. For example, the server device 10 can be implemented as a server that provides the creation supporting function described above in a on-premise. Besides, the server device 10 can provide the creation supporting function described above as a cloud service by implementing in a form of platform as a service (PaaS) type or software as a service (SaaS) type application.

The server device 10 can be connected to a client terminal 30 through a network NW in a communication-enabled manner as illustrated in FIG. 1. For example, the network NW may be a communication network of an arbitrary type, such as the Internet and a local area network (LAN), regardless of wired or wireless communications. In FIG. 1, an example in which a single unit of the client terminal 30 is connected to a single unit of the server device 10 is illustrated, but it does not prevent connection of the client terminal 30 of an arbitrary number of units.

The client terminal 30 corresponds to an example of a computer that is provided with the creation supporting function described above. For example, the client terminal 30 may be implemented by a portable terminal device, such as a personal computer, a smartphone, a tablet terminal, and a wearable terminal.

In FIG. 1, an example as a usage scene in which the server device 10 provides the creation supporting function described above to the client terminal 30 is illustrated, but this is only one example. For example, the creation supporting function described above may be provided in a stand-alone manner by an application that operates on the client terminal 30 causing the client terminal 30 to perform processing corresponding to the creation supporting function described above.

### Measure Flow

In the following, a measure flow is exemplified just as an example of a workflow, and a person is exemplified just as an example of an object to be distributed by the workflow.

The "measure flow" herein is to assign persons subject to the measure in various fields, such as medicine, nursing, and administration, to a service to achieve a goal of the measure or the like. For example, the measure flow includes elements, such as "conditional branch" and "intervention".

Out of these, "conditional branch" corresponds to an element of distributing persons subject to the measure. Moreover, "intervention" corresponds to an element in which a service or an action to achieve a goal of the measure are defined.

The service that is thus distributed in the measure flow can be provided by a medical service provider. For example, the medical service provider may include medical workers, such as doctor and nurse, and also government service providers, such as public health nurse and public health promotion department.

Accordingly, when a measure flow is to be planned, a point of view whether human resources such as medical service providers can be appropriately distributed to services to which persons are distributed by the measure flow is important.

### Importance of Comparison

A measure planning process includes [A] Discovery and Review of Problems, [B] Planning and Operation of Measure, [C] Verification and Assessment of Effect, and the like. Out of these, planning of a measure corresponds to [B] Planning and Operation of Measure. Furthermore, [B] Planning and Operation of Measure includes processes, such as formulation of measure candidates, analysis, comparison, and determination of measures, and the like.

Planning of such measure candidates include four requirements. For example, as the first one, using an existing measure substitute plan as it is exemplified. As the second one, diversion of a measure that has taken in another region as a measure substitute plan can be exemplified. As the third one, planning by referring to a list of general policy measures can be exemplified. As the fourth one, planning a completely new policy substitute can be exemplified.

In addition to these four requirements, the importance of comparing an originally planned measure flow and a measure flow of an existing measure has been increasing because it is obvious that importance is given to whether a similar measure has been taken in past for planning a measure from a viewpoint of administrative implementability.

Hereinafter, the originally planned measure flow can be denoted as "original flow", and the measure flow of an existing measure can be denoted as "comparative flow".

### One Aspect of Problem

As explained in the above section of problems, because conventional techniques represented by the energy-saving-evaluation supporting device described above and the like are to only search for related measures that can be related to a measure to be planned, there is an aspect that it is not necessarily possible to provide information contributing to judgement on excess and deficiency of resources.

For example, a measure flow has an aspect that persons subject to measures are distributed to a service or the like to achieve a goal of the measures through conditional branches defined in the measure flow. However, the related measures described above only resemble in scale and kind of facilities of a building to the measure to be planned. Even if a measure flow of such a related measure is presented, it is difficult to judge excess and deficiency of resources.

### One Aspect of Problem Solving Approach

Accordingly, as a part of the creation supporting function according to the present embodiment, a display function of visualizing and displaying a distribution state of persons that are distributed to respective services in an original flow and a comparison flow is provided.

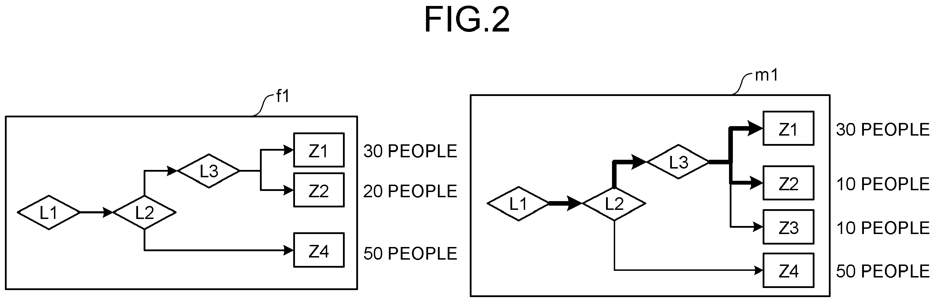

FIG. 2 is a diagram illustrating a display example of the original flow and the comparison flow. In FIG. 2, just as one example of the comparison flow, a comparison flow m1 similar in structure to an original flow f1 is illustrated. As illustrated in FIG. 2, according to the display function described above, the original flow f1 and the comparison flow m1 are displayed. For example, in the original flow f1, the number of persons to be distributed by conditional branches L1, L2, and L3 for each of services Z1, Z2, and Z4 is displayed. On the other hand, in the comparison flow m1, the number of persons to be distributed by the conditional branches L1, L2, and L3 is displayed for each of services Z1, Z2, Z3, and Z4.

It is found that the services Z1, Z2, and Z4 are common between the original flow f1 and the comparison flow m1. Furthermore, the number of persons distributed to the respective services Z1 and Z4 is common, and 10 persons are common in the number of persons distributed to the service Z2 also. Thus, the number of persons that is affected by a difference between the original flow f1 and the comparison flow m1 is 10 persons out of total 100 persons and, therefore, it is found that the comparison flow m1 is similar measure flow to the original flow f1.

It can be said that the comparison flow m1 like this is appropriate as an index to judge whether human resources can be distributed appropriately to the number of persons distributed to the services Z1, Z2, and Z4 of the original flow f1.

By comparing the original flow f1 and the comparison flow m1, just as one example, it is possible to grasp excess and deficiency of human resources to be distributed to a service from a difference in the number of persons distributed to the same service.

For example, in a service in which the number of persons distributed in the original flow f1 is too large compared to the number of persons distributed in the comparative flow m1, a possibility that a load on a medical service provider assigned to the service increases or exceeds the capacity can be grasped. Moreover, in a service in which the number of persons distributed in the original flow f1 is too small compared to the number of persons distributed in the comparative flow m1, it is possible to grasp that there is surplus human resources to be assigned to the service.

Therefore, according to the display function according to the present embodiment, information contributing to judgment on excess and deficiency of resources can be provided.

### Configuration of Server Device 10

Next, a functional configuration example of the server device 10 according to the present embodiment will be explained. FIG. 1 schematically illustrates blocks relating to the display function included in the server device 10. As illustrated in FIG. 1, the server device 10 includes a communication control unit 11, a storage unit 13, and a control unit 15. Note that functional units relating to the display function described above are only selectively illustrated in FIG. 1, and functional units other than those illustrated may also be provided in the server device 10.

The communication control unit 11 is a functional unit that controls communication of the client terminal 30 and the like with other devices. Just as one example, the communication control unit 11 can be implemented by a network interface card, such as LAN card. As one aspect, the communication control unit 11 accepts a search request for a comparison flow from the client terminal 30, or outputs display data in which a state in which persons are distributed to a service in the original flow and the comparison flow and the like to the client terminal 30.

The storage unit 13 is a functional unit that stores various kinds of data. Just as one example, the storage unit 13 is implemented by an internal, external, or auxiliary storage of the server device 10. For example, the storage unit 13 stores resident data 13A and flow data 13B. Explanation of the resident data 13A and the flow data 13B will be given together with a stage at which reference, creation, or registration is performed.

The control unit 15 is a functional unit that performs overall control of the server device 10. For example, the control unit 15 can be implemented by a hardware processor. Other than this, the control unit 15 may be implemented by hardwired logic. As illustrated in FIG. 1, the control unit 15 includes an accepting unit 16, a searching unit 17, and a display unit 18.

The accepting unit 16 is a processing unit that accepts various kinds of request from the client terminal 30. Just as one example, the accepting unit 16 can accept a search request for the comparison flow from the client terminal 30.

When such a request is to be accepted, the accepting unit 16 can accept designation of the original flow used for the search for the comparison flow also. As one aspect, the accepting unit 16 can accept the original flow from the client terminal 30 through the network NW. As another aspect, the accepting unit 16 can receive designation of the original flow from a measure flow included in the flow data 13B stored in the storage unit 13. As still another aspect, the accepting unit 16 can accept designation from a measure flow stored in a database server or a file system not illustrated.

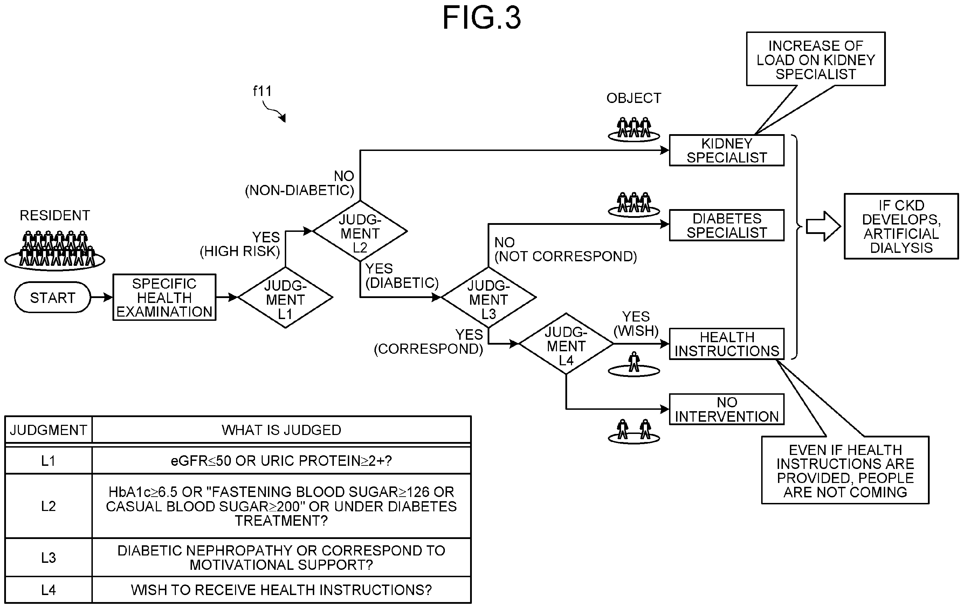

FIG. 3 is a diagram illustrating an example of the original flow. In FIG. 3, as one example of the original flow, a measure flow relating to diagnosis flow of chronic kidney disease (CKD) is illustrated.

Such a measure flow can be created by a special committee based on a nephropathy worsening-prevention measure. For example, the special committee may include a kidney specialist, a diabetes specialist, a public health nurse, and a public health promotion department/health policy department of a city.

As illustrated in FIG. 3, a measure flow f11 distributes residents to services "kidney specialist", "diabetes specialist", "public health nurse", or "no intervention" through the conditional branches L1, L2, L3, and L4.

For example, when judgment of the conditional branch L1 is YES and the judgment of the conditional branch L2 is NO (non-diabetic), a resident is distributed to the service by a kidney specialist. Moreover, when judgment of the conditional branch L1 is YES, judgment of the conditional branch L2 is YES (diabetic), and judgment of the conditional branch L3 is NO, a resident is distributed to the service by a diabetes specialist. Furthermore, when judgment of the conditional branch L1 is YES, judgment of the conditional branch L2 is YES (diabetic), judgment of the conditional branch L3 is YES, and judgment of the conditional branch L4 is YES, a resident is distributed to the service of health instructions. Moreover, when judgment of the conditional branch L1 is YES, judgment of the conditional branch L2 is YES (diabetic), judgment of the conditional branch L3 is YES, and judgment of the conditional branch L4 is NO, it is determined as no intervention, and distribution to a specific service is not performed.

The measure flow f11 as described has a problem that a load of a kidney specialist increases or that a resident does not come even though health instructions are provided. On the other hand, in planning of the measure flow f1, from the viewpoint of implementability, it would be better to implement by a measure as close as possible to an existing measure by a trouble-saving method.

The searching unit 17 is a processing unit that searches for a comparison flow that is similar to the original flow. Just as one example, when the search request described above is accepted by the accepting unit 16, the searching unit 17 acquires resident data, an original flow, and M pieces of comparison flows.

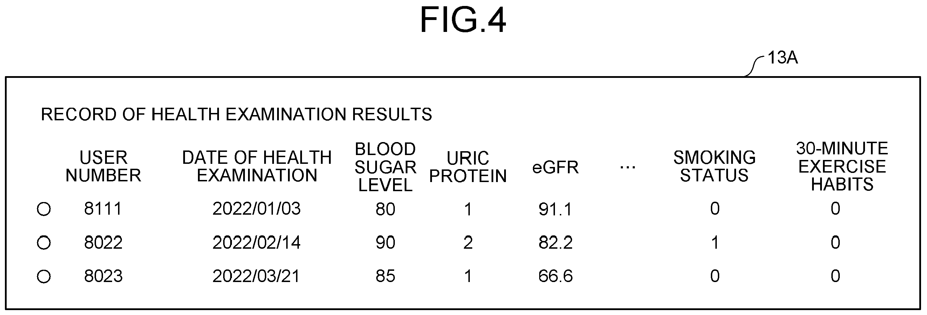

More specifically, the searching unit 17 acquires the resident data 13A stored in the storage unit 13. The resident data 13A is data in which personal information relating to a resident is associated with each resident. FIG. 4 is a diagram illustrating an example of the resident data 13A. In FIG. 4, a result of a health examination is illustrated as personal information of a resident. As illustrated in FIG. 4, the resident data 13A can include items of user number, date of examination, blood sugar level, uric protein, eGFR, smoking status, 30-minutes exercise habits, and the like. "User umber" herein corresponds to one example of identification information of a resident corresponding to a user. Moreover, "date of examination" indicates a date on which a health examination of the resident is carried out. Moreover, "blood sugar level", "eGFR", "smoking status", and "30-minutes exercise habits" corresponds to examination items checked in a health examination and, for example, while results of "blood sugar level", "eGFR", and "smoking status" are recorded in numerical values, for "smoking status" and "30-minutes exercise habits" are recorded in a binary value flag corresponding to true or not true. By thus performing judgment of the conditional branches of the measure flow or the comparison flow based on the result of such a health examination, residents can be distributed to a service of respective flows of the measure flow and the comparison flow.

Furthermore, the searching unit 17 acquires an original flow that is designated by the search request described above. Moreover, the searching unit 17 acquires M (positive integer) pieces of comparison flows from among measure flows included in the flow data 13B stored in the storage unit 13. The flow data 13B may be a database in which measure flows are collected. In the flow data 13B as described, measure flows that have planned in past can be collected. For example, the flow data 13B may include a measure flow that has been created by a department other than a department to which a user of the client terminal 30, that is, a measure planner being an example of a user of the display function described above belongs, a measure flow that has been created in a region other than the region to which the measure planner belongs, and the like. When a comparison flow is acquired from the flow data 13B as described, the comparison flow can be acquired by acquiring the entire measure flows included in the flow data 13B, or by narrowing down to a measure flow of a similar measure that resembles to the measure of the original flow by using an existing technique.

Having acquired the resident data, the original flow and M pieces of the comparison flows, the searching unit 17 performs following processing for the number of times corresponding to K people of residents, for each of the M pieces of the comparison flows. That is, the searching unit 17 identifies a route on a flow to which a resident k is distributed by conditional branches for each of the original flow f and the comparison flow m, by collating an entry of the resident k in the resident data 13A with conditional branches of the original flow f and the comparison flow m.

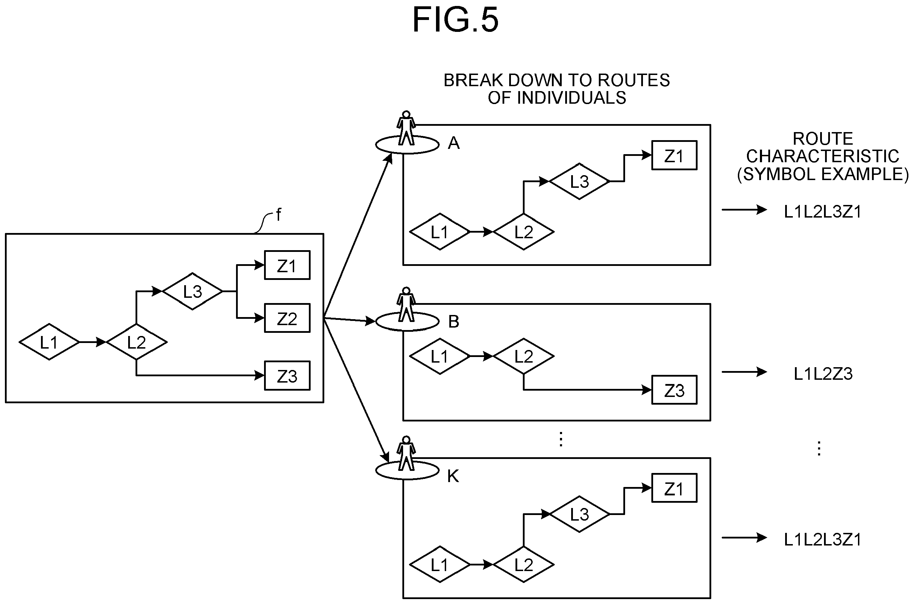

FIG. 5 is a diagram illustrating an example of route identification. In FIG. 5, the original flow f is illustrated as an example of a measure flow, and A, B, ..., and K are illustrated as an example of the residents.

As illustrated in FIG. 5, elements of the original flow f, such as a conditional branch and a service, are converted into symbols. Just as one example, by hashing text included in an element of the original flow f, the element of the original flow f can be converted in to a symbol.

After the element of the original flow f is thus converted into a symbol, the elements of the original flow f are searched sequentially from an upstream side of the original flow f. When the element is a conditional branch, a direction of a branch destination is identified based on whether information relating to a condition set to the conditional branch, for example, a numerical value or a flag, satisfies the condition of the conditional branch out of a result of health examination of the resident k. By repeating the search as described until it reaches an end point of the original flow f, a sequence of elements along which the resident k flows on the original flow f is acquired as a symbol string.

For example, in a case of the resident A, a symbol string "L1L2L3Z1" is acquired. Furthermore, in the example of the resident B, a symbol string "L1L2Z3" is acquired. Moreover, in the example of the resident K, a symbol string "L1L2L3Z1" is acquired.

In FIG. 5, an example of identifying a route on a flow to which the resident k is distributed based on the conditional branch by the original flow f is exemplified, but a route on a flow to which the resident k is distributed based on the conditional branch by the comparison flow can also be identified similarly.

Returning back to explanation of FIG. 1, the searching unit 17 includes a counting unit 17A and a calculating unit 17B.

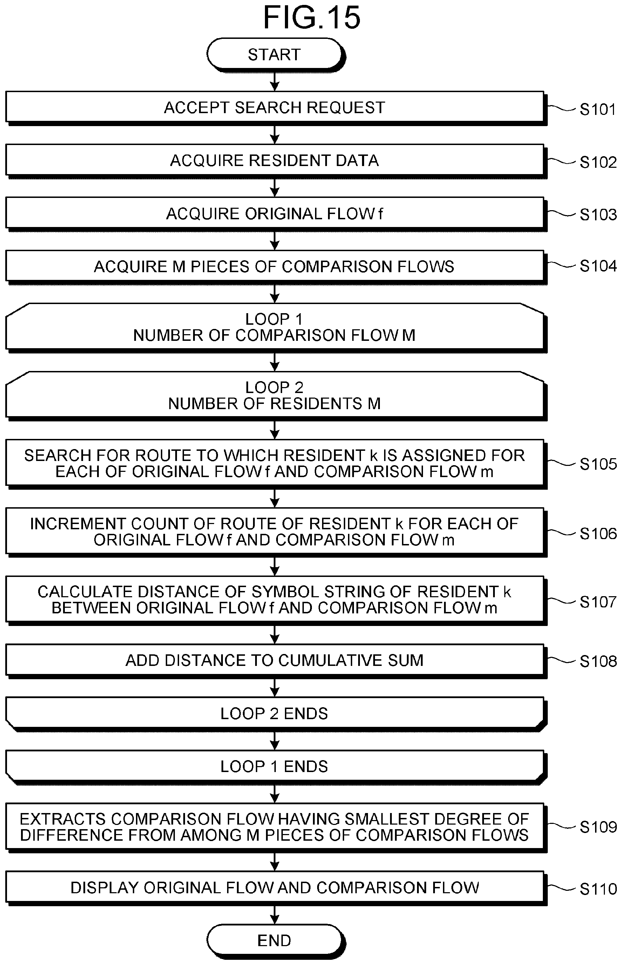

The counting unit 17A is a processing unit that counts the number of residents distributed to respective routes in the original flow and the comparison flow. For example, in the example illustrated in FIG. 15, out of three routes of the symbol string "L1L2L3Z1", a symbol string "L1L2L3Z2", and the symbol string "L1L2Z3", a counter to count the number of residents in a route to which the residents are distributed is incremented. In the example of the resident "A", the counter of the route corresponding to the symbol string "L1L2L3Z1" is incremented. Furthermore, in the example of the resident "B", the counter of the route corresponding to the symbol string "L1L2Z3" is incremented. Moreover in the example of the resident "K", the counter of the route corresponding to the symbol string "L1L2L3Z1" is incremented.

The calculating unit 17B is a processing unit that calculates a degree of difference between the original flow f and the comparison flow m. Just as one example, the calculating unit 17B calculates a distance between symbol strings corresponding to routes to which the resident k is distributed in the original flow f and the comparison flow m. By thus calculating a statistical value, for example, an average value, a total value, and the like, of the distance calculated for each of K people of residents, a degree of difference between the original flow f and the comparison flow m can be calculated.

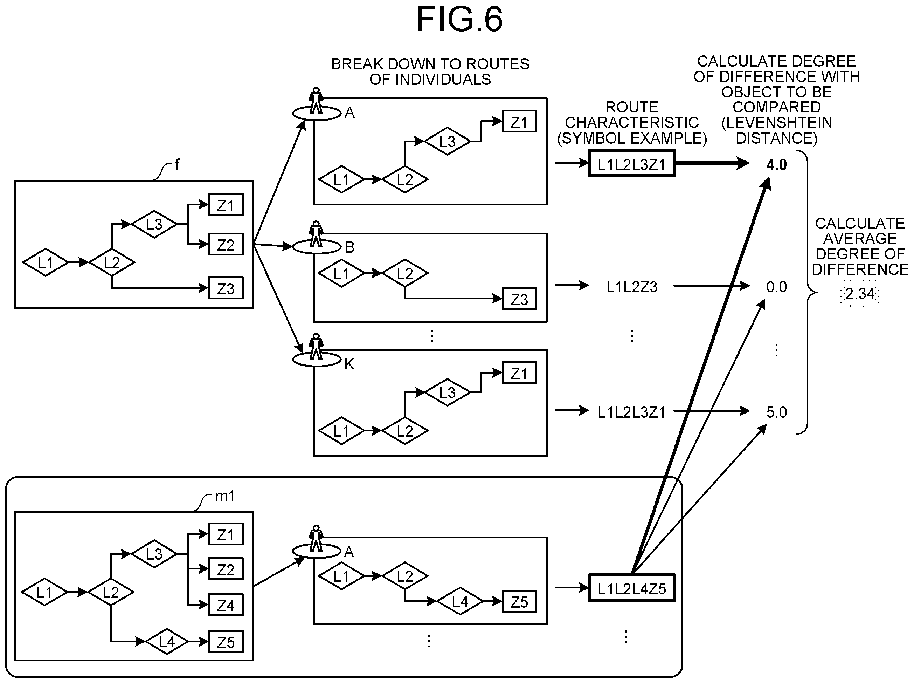

FIG. 6 is a schematic diagram illustrating a calculation example of the degree of difference. In FIG. 6, as an example of the measure flow, the original flow f illustrated in FIG. 5 is illustrated, and the comparison flow m1 is illustrated. Furthermore, in FIG. 6, as an example of the residents, A, B, ..., K are illustrated.

For example, in the case of the resident A, a distance between the symbol string "L1L2L3Z1" corresponding to the route to which A is distributed by the original flow f and a symbol string "L1L2L4Z5" corresponding to a route to which A is distributed by the comparison flow m1 is calculated.

For calculation of a distance between these symbol strings, just as one example, the Levenshtein distance that defines a distance between two character strings can be used. That is, by repeating editing processes, such as "insertion", "deletion", and "replacement", with respect to one of character strings, the minimum number of times until it is converted into the other character string is to be the Levenshtein distance.

Just as one example, when "insertion" and "deletion" are permitted but "replacement" is prohibited out of the editing processes, a distance of symbol strings between the original flow f and the comparison flow m of the resident A is acquired as follows.

That is, as the first editing process, a symbol "L4" is inserted to the symbol string "L1L2L3Z1". Thus, it is edited to a symbol string "L1L2L3Z1L4". Next, as the second editing process, a symbol "Z1" is deleted from the symbol string "L1L2L3Z1L4". Thus, it is edited to a symbol string "L1L2L3L4". Next, as the third editing process, a symbol "L3" is deleted from the symbol string "L1L2L3L4". Thus, it is edited to a symbol string "L1L2L4". Finally, as the fourth editing process, a symbol "Z5" is inserted to the symbol string "L1L2L4". Thus, it is edited to the symbol string "L1L2L4Z5". Because the symbol string "L1L2L3Z1" is edited to the symbol string "L1L2L4Z5" by the four times of editing process as described above, the Levenshtein distance is calculated as "4.0". If the editing process of "replacement" is permitted, the minimum editing distance is to be "2".

As described, for each of the K people of residents, a distance between a symbol string of a distributed route by the original flow f and a symbol string of a distributed route by the comparison flow m1 is calculated. Besides, by calculating a statistic value, for example, an average value, of the distance of symbol strings calculated for each of the K people of residents, a degree of difference "2.34" is acquired.

According to such a degree of difference, a flow of persons in the original flow f and the comparison flow m is expressed in a symbol string, and a degree of difference is calculated from a distance of the symbol string. Therefore, not only a similarity in a graph structure between the original flow f and the comparison flow m, but also a similarity in a flow of persons between the original flow f and the comparison flow m can be reflected in a value of the degree of difference.

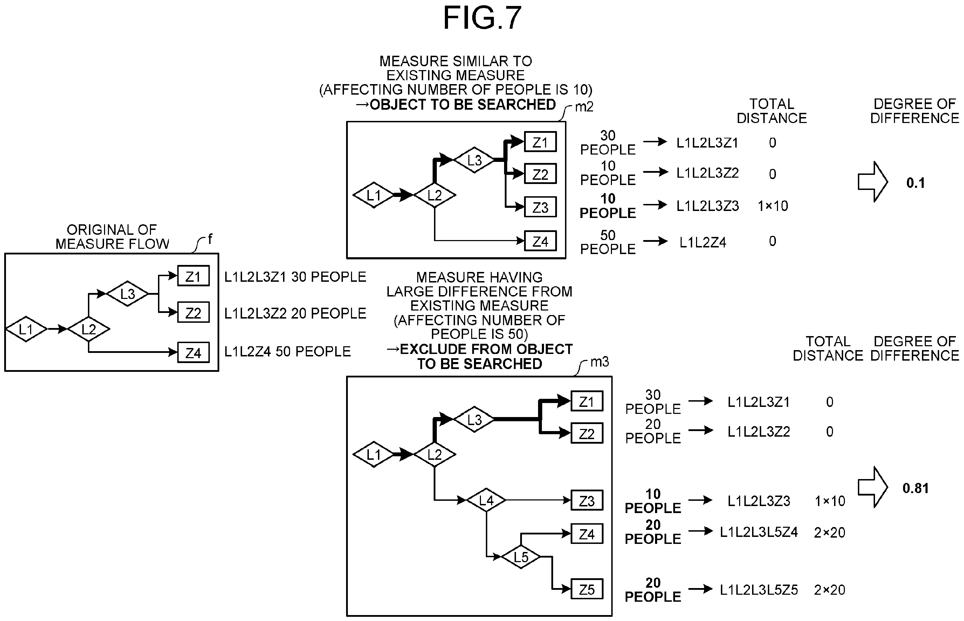

FIG. 7 is a schematic diagram illustrating a calculation example of the degree of difference. In FIG. 7, as an example of the measure flow, the original flow f illustrated in FIG. 5 is illustrated, and a comparison flow m2 and a comparison flow m3 are illustrated.

As illustrated in FIG. 7, between the original flow f and the comparison flow m2, routes of all of 100 people of residents are compared. In this case, between the original flow f and the comparison flow m2, it is duplicated in a point that 80 people of the residents take the symbol string "L1L2L3Z1" and the symbol string "L1L2L4" as the route, and 10 people of the residents take the symbol string "L1L2L3Z2" as the route. Because the distance of these 90 people is to be 0, the distance of the remaining 10 people affects the degree of difference. That is, a total value "10" of distance "1" between the symbol string "L1L2L3Z2" of the original flow f and a symbol string "L1L2L3Z3" of the comparison flow m2 of 10 people is divided by 100 people, to acquire the degree of difference "0.1".

Meanwhile, routes of all 100 people of the residents are compared between the original flow f and the comparison flow m3. In this case, between the original flow f and the comparison flow m3, it is duplicated in a point that 50 people of the residents take the symbol string "L1L2L3Z1" and the symbol string "L1L2L3Z2" as the route. Because the distance of these 50 people is to be 0, the distance of the remaining 50 people affects the degree of difference. That is, a total value "10" of distance "1.0" between the symbol string "L1L2Z4" of the original flow f and a symbol string "L1L2L4Z3" of the comparison flow m3 of 10 people is acquired. Furthermore, a total value "40" of distance "2.0" between the symbol string "L1L2Z4" of the original flow f and a symbol string "L1L2L4L5Z4" of the comparison flow m3 of 20 people is acquired. Moreover, a total value "40" of distance "2.0" between the symbol string "L1L2Z4" of the original flow f and a symbol string "L1L2L4L5Z5" of the comparison flow m3 of 20 people is acquired. A total value "90" of the distances of these 50 people is divided by 100 people, to acquire the degree of difference "0.9".

As described, because the original flow f and the comparison flow m2 have most part of the flow of person, that is, 90% in common, the degree of difference can be calculated low. On the other hand, between the original flow f and the comparison flow m3, because a flow of 50 people, which is a half of the people, is different, the degree of difference can be calculated high.

Furthermore, in the routes included in the comparison flow m, if a route, a frequency of a flow of person of which is equal to or lower than a threshold, or a route, a frequency of a flow of person of which exceeds a capacity set to a service is included, the comparison flow m can be excluded from objects to be displayed.

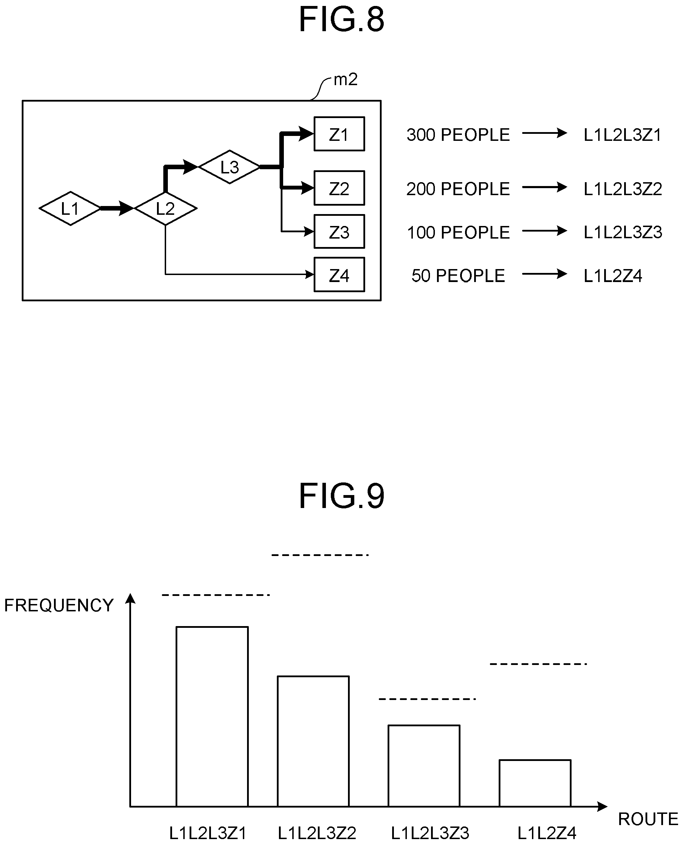

FIG. 8 is a diagram illustrating an example of the comparison flow. FIG. 9 is a diagram illustrating an example of a relationship between a route and a frequency. The comparison flow m2 illustrated in FIG. 8 includes four routes of the symbol string "L1L2L3Z1", the symbol string "L1L2L3Z2", the symbol string "L1L2L3Z3", and the symbol string "L1L2Z4". The frequency at which the residents are distributed to these four routes are as illustrated in FIG. 9. In FIG. 9, for each of four services Z1, Z2, Z3, and Z4, a capacity of the service is indicated with a broken line. In the example in FIG. 9, there is no route, the frequency at which residents are distributed of which is nearly 0, and there is no route, the frequency at which residents are distributed of which exceeds the capacity, either. Therefore, the comparison flow m2 is not excluded from objects to be displayed.

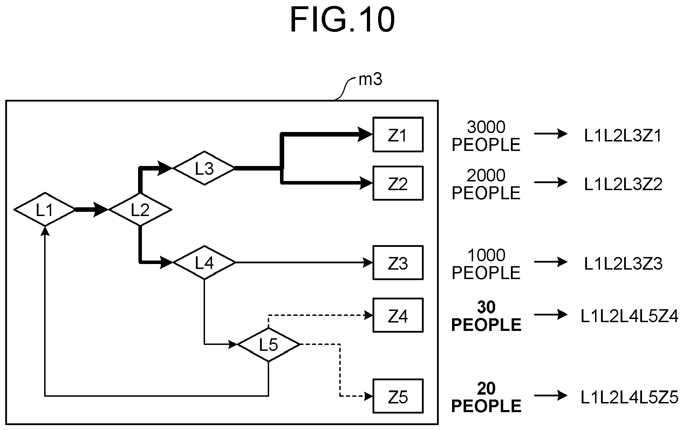

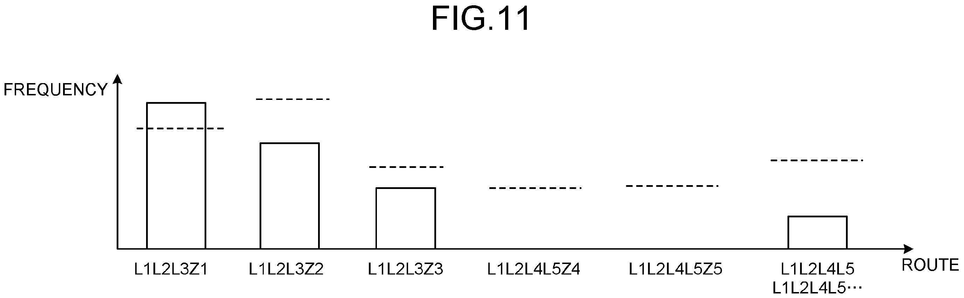

FIG. 10 is a diagram illustrating an example of the comparison flow. FIG. 11 is a diagram illustrating an example of a relationship between a route and a frequency. The comparison flow m3 illustrated in FIG. 10 includes five routes of the symbol string "L1L2L3Z1", the symbol string "L1L2L3Z2", the symbol string "L1L2L3Z3", the symbol string "L1L2L4L5Z4", and the symbol string "L1L2L4L5Z5". The frequency at which the residents are distributed to these five routes are as illustrated in FIG. 11. In FIG. 11, for each of five services Z1, Z2, Z3, Z4 and Z5, a capacity of the service is indicated with a broken line. In the example in FIG. 11, there is the route of the symbol string "L1L2L3Z1" exceeds the capacity. Furthermore, the frequencies at which residents are distributed to the routes of the symbol string "L1L2L4L5Z4" and the symbol string "L1L2L4L5Z5" are nearly 0. Therefore, the comparison flow m3 can be excluded from objects to be displayed.

Moreover, a flow of persons in the original flow f and the comparison flow m is expressed in a symbol string, and a degree of difference is calculated from a distance of the symbol string. Accordingly, the degree of difference is calculated high as a degree of difference in structure between the original flow f and the comparison flow m becomes high and, consequently, a possibility that the comparison flow m in which the degree of difference in structure is high is selected as an object to be displayed can be reduced.

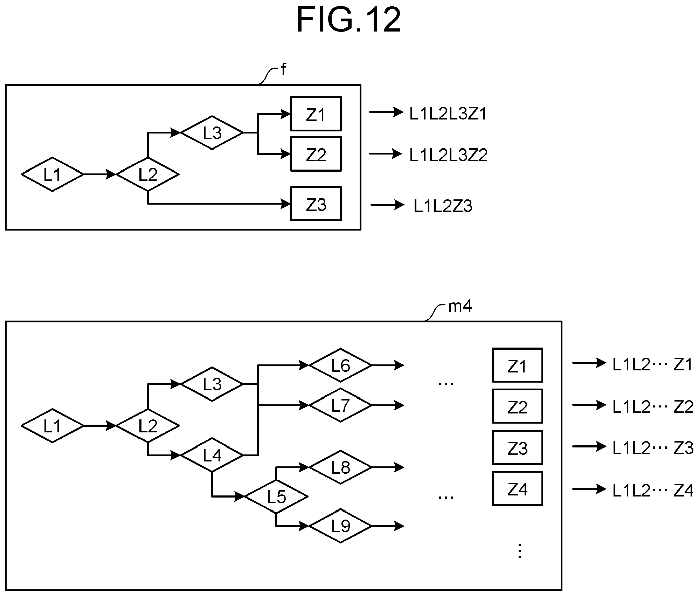

FIG. 12 is a diagram illustrating an example of the original flow and the comparison flow. As illustrated in FIG. 12, the original flow f includes routes of the symbol string "L1L2L3Z1", the symbol string "L1L2L3Z2", and the symbol string "L1L2L3Z3". On the other hand, the comparison flow m4 includes routes of the symbol string "L1L2...Z1", the symbol string "L1L2...Z2", the symbol string "L1L2...Z3", the symbol string "L1L2...Z4", and the like. When the original flow f and the comparison flow m4 are compared with each other, the symbol strings of the routes included in the comparison flow m4 is longer than the symbol strings of the routes included in the original flow f. Therefore, the degree of difference between the original flow f and the comparison flow m4 becomes high. Therefore, a possibility that the comparison flow m4 is selected as an object to be displayed can be reduced.

After the degree of difference between the original flow f and the comparison flow m is thus calculated for each of the M pieces of comparison flows, the searching unit 17 extracts, just as one example, a comparison flow, the degree of difference of which is the lowest among the M pieces of comparison flows as an object to be displayed.

Herein, just as one example, a case in which a comparison flow having the lowest degree of difference is extracted as an object to be displayed is exemplified, it is not limited thereto. For example, the predetermined number of comparison flows can be extracted as an object to be displayed sequentially from the lowest degree of difference. Other than this, comparison flows can be sorted in ascending order of the degree of difference, and designation of a comparison flow to be an object to be displayed can be accepted from a list of the sorted comparison flows.

The display unit 18 is a processing unit that performs various kinds of displays to the client terminal 30. Just as one example, the display unit 18 generates display data in which a distribution state of residents to be distributed to respective services is visualized for each of the original flow f designated in the search request described above and the comparison flow m extracted by the searching unit 17, and displays it on the client terminal 30.

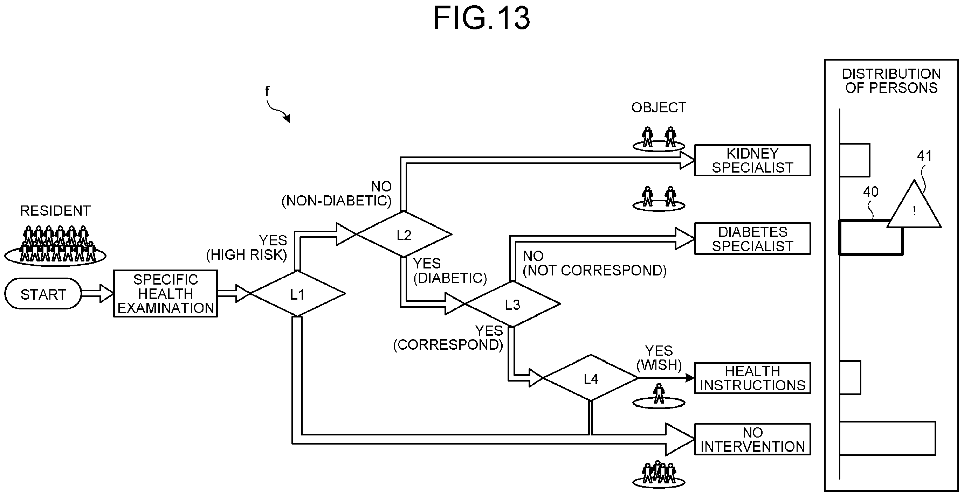

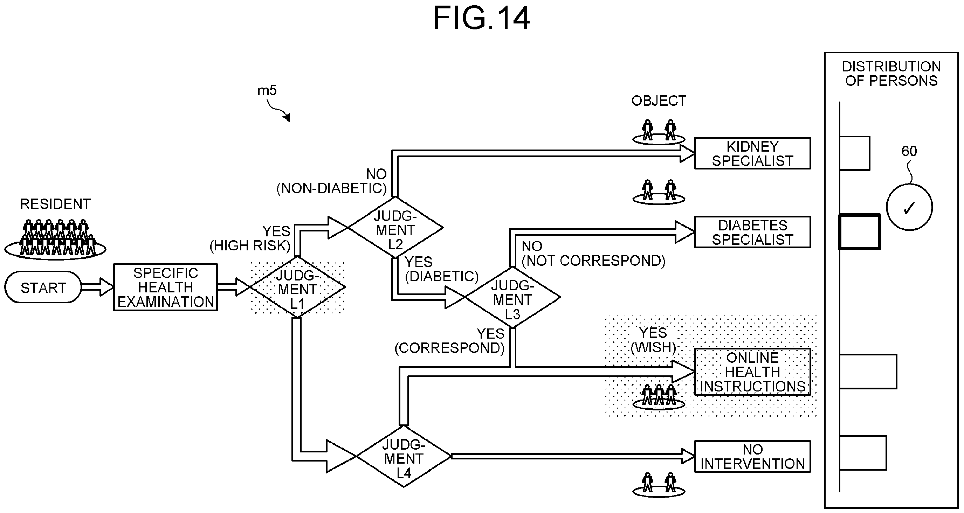

FIG. 13 is a diagram illustrating a display example of the original flow. FIG. 14 is a diagram illustrating a display example of the comparison flow. Supplementary explanation about the conditional branch in FIG. 13 will be given herein. To the conditional branch L1 illustrated in FIG. 13, a condition whether eGFR is lower than 50 or whether uric protein is 2++ or higher is set. Moreover, to the conditional branch L2 illustrated in FIG. 13, a condition whether it is diabetic is set. Furthermore, to the conditional branch L3 illustrated in FIG. 13, a condition whether already seeing a doctor is set. Furthermore, to the conditional branch L4 illustrated in FIG. 13, a condition whether health instructions are wished is set.

As illustrated in FIG. 13 and FIG. 14, in the original flow f and the comparison flow m5, the numbers of residents to be distributed to the respective services are displayed in a bar chart. A case in which the number of residents are displayed in a bar chart is exemplified herein, but the number of residents may be displayed in a numerical value, may be displayed in level, or may be displayed in a symbol, such as icons, and it is not limited to a specific display form. By such a display of the number of people per service, a difference in the number of residents to be distributed to the same service between the original flow f and the comparison flow m5 can be visualized and, therefore, information contributing to judgment of excess and deficiency of human resources, such as medical service providers, can be provided.

Furthermore, as illustrated in FIG. 13 and FIG. 14, in the original flow f and the comparison flow m5, a display form of an edge connecting between elements of nodes is changed according to the number of residents to be distributed to the edge. For example, while as the number of residents distributed to an edge increases, the width of the edge is displayed thicker, as the number of residents distributed to an edge decreases, the width of the edge is displayed thinner. By such a distinguished display of edges, an excessive flow of persons and a deficient flow of persons can be grasped in a unit of edge and, therefore, information contributing to judgment of adequacy of a conditional branch, in addition to the judgment of excess and deficiency of human resources, can be provided.

Moreover, as illustrated in FIG. 13, in the original flow f, a service for which a difference in the number of residents between the original flow f and the comparison flow m5 is equal to or larger than a threshold, or a service for which the number of residents exceeds the capacity is displayed in a display form different from a display form of other services. For example, as a service by a diabetes specialist illustrated in FIG. 13, an intensified display 40 of an outline of a bar is performed, or a cautionary alert display 41 is performed. In contrast to the intensified display 40 or the alert display 41 of the original flow f, when the same service does not exceed the capacity in the comparison flow m5, an OK mark 60 indicating that distribution of residents satisfies specialist resource is displayed for the service.

Furthermore, as illustrated in FIG. 14, in the comparison flow m5, out of elements corresponding to each other in the original flow f and the comparison flow m5, an element in which text defining the element does not completely coincide, but the similarity of the text is equal to or higher than a threshold is displayed in a display form different from other elements. For example, an element corresponding to the conditional branch L1 of the original flow f and an element corresponding to health instructions are displayed in hatching. By such a hatching display of the comparison flow m5, a partial difference of the comparison flow m5 from the original flow f is presented and, therefore, an improvement with respect to the original flow f can be indicated. For example, the presentation of the difference from the conditional branch L1 of the original flow f can provide a motivation to recognize the importance of suppressing residents that can be seen by a specialist. Moreover, as the service "health instructions" of the original flow f and a service "online health instructions" of the comparison flow m5 are compared, a motivation of recognizing the importance of putting health instructions online from the viewpoint of achieving health instructions to more people can be provided.

### Flow of Processing

FIG. 15 is a flowchart illustrating a procedure of the display processing. Just as one example, as illustrated in FIG. 15, when a search request for a comparison flow is accepted from the client terminal 30 (step S101), it can be started.

Subsequently, the searching unit 17 acquires the resident data 13A stored in the storage unit 13 (step S102). Furthermore, the searching unit 17 acquires the original flow designated in the search request described above (step S103). Moreover, the searching unit 17 acquires M pieces of the comparison flow from among measure flows included in the flow data 13B that is stored in the storage unit 13 (step S104).

The processing at these step S102 to step S104 are not always to be performed in order of step numbers, and may be performed irrespectively of order or in parallel.

The searching unit 17 performs loop processing 1 and loop processing 2 in which processing from step S105 described below to step S108 described below is repeated for the number of times corresponding to K people of residents for each of the M pieces of comparison flow. The processing from step S105 to step S108 described below is not necessarily required to be performed in repetition, but may be performed in parallel.

That is, the searching unit 17 collates an entry of the resident k in the resident data 13A acquired at step S102 with a conditional branch of the original flow f and the comparison flow m. Thus, the searching unit 17 identifies a route on a flow to which the resident k is distributed by the conditional branch for each of the original flow f and the comparison flow m (step S105).

Subsequently, the counting unit 17A increments a counter of the number of residents corresponding to the route on the flow tow which the resident k is distributed at step S105 for each of the original flow f and the comparison flow m (step S106).

Thereafter, the calculating unit 17B calculates a distance of a symbol string corresponding to the route to which the resident k is distributed for each of the original flow f and the comparison flow m (step S107). Besides, the calculating unit 17B accumulates the distance of a symbol string of the resident k calculated at step S107 to a total value to this point (step S108).

By repeating the loop processing 2 as described, a route on the flow to which the resident k is distributed is acquired for each of the resident k for each of the original flow f and the comparison flow m, and the total value of distances of symbol strings of all of K people of residents can be acquired. For example, by dividing the total value by K people, the degree of difference of the original flow f and the comparison flow m can be calculated.

Furthermore, by repeating the loop processing 1, the degree of difference of the original flow f and the comparison flow m is calculated for each of the M pieces of the comparison flow.

Thereafter, the searching unit 17 extracts a comparison flow having the smallest degree of difference from among the M pieces of the comparison flow (step S109). The display unit 18 displays display data in which a distribution state of residents to be distributed to respective services is visualized on the client terminal 30 for each of the original flow f designated in the search request described above and the comparison flow m extracted at step S109 (step S110), and the processing is ended.

### One Aspect of Effect

As described above, the server device 10 according to the present embodiment visualizes a distribution state in which persons are distributed to respective services in the original flow and the comparison flow, and displays it. Therefore, according to the server device 10 according to the present embodiment, a difference in the number of persons distributed to the same service between the original flow f and the comparison flow m5 can be visually presented and, therefore, information contributing to judgment of excess and deficiency of resources can be provided.

### Second Embodiment

Embodiments relating to the disclosed device has so far been explained, but other than the embodiments described above, the present invention may be implemented in various other forms. In the following, other embodiments included in the present invention will be explained.

### Application Other than Measure Flow

In the first embodiment described above, a measure flow has been exemplified as an example of a workflow, but this is only one example, and the processing illustrated in FIG. 15 can be applied similarly to general workflows other than the measure flow. Furthermore, a person has been exemplified as an object to be applied to a workflow, but the object may be other things than a person, and may be, for example, a manufactured product.

### Distribution and Integration

Moreover, the respective components of the respective devices illustrated are not necessarily required to be configured physically as illustrated. That is, specific forms of distribution and integration of the respective devices are not limited to the ones illustrated, and all or some thereof can be configured to be distributed or integrated functionally or physically in arbitrary units according to various kinds of loads, use conditions, and the like. For example, the accepting unit 16, the searching unit 17, or the display unit 18 may be connected to the server device 10 through a network as an external device. Moreover, the accepting unit 16, the searching unit 17, and the display unit 18 may be arranged respectively in separate devices and connected through a network so as to cooperate with each other, and to implement the functions of the server device 10. Furthermore, an entirety or a portion of the resident data 13A or the flow data 13B stored in the storage unit 13 may be held respectively in separate devices, and those may be connected through a network to cooperate with each other, to implement the functions of the server device 10.

### Hardware Configuration

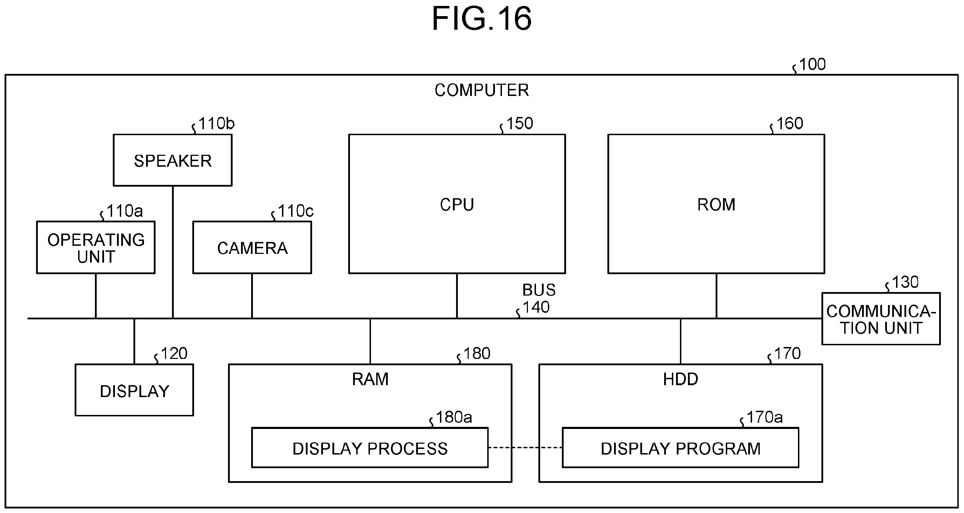

Furthermore, the respective processing explained in the embodiments described above can be implemented by executing a program prepared in advance by a computer, such as a personal computer and a workstation. In the following, an example of a computer that executes a display program having functions similar to the first embodiment and the second embodiment will be explained by using FIG. 16.

FIG. 16 is a diagram illustrating a hardware configuration example. As illustrated in FIG. 16, a computer 100 includes an operating unit 110a, a speaker 110b, a camera 110c, a display 120, and a communication unit 130. Furthermore, the computer 100 includes a central processing unit (CPU) 150, a read-only memory (ROM) 160, a hard disk drive (HDD) 170, and a random access memory (RAM) 180. The respective components 110 to 180 are connected to one another through a bus 140.

The HDD 170 stores, as illustrated in FIG. 16, a display program 170a that exerts functions similar to the accepting unit 16, the searching unit 17, and the display unit 18 illustrated in the first embodiment described above. This display program 170a may be integrated or separated similarly to the respective components of the accepting unit 16, the searching unit 17, and the display unit 18 illustrated in FIG. 1. That is, as long as data to be used for processing is stored in the HDD 170, the entire data illustrated in the first embodiment described above is not necessarily required to be stored in the HDD 170.

In such an environment, the CPU 150 reads out the display program 170a from the HDD 170, and expands on the R_AM 180. As a result, the display program 170a functions as a display process 180a as illustrated in FIG. 16. This display process 180a expands various kinds of data read from the HDD 170 in an area allocated to the display process 180a out of a storage area included in the R_AM 180, and performs various kinds of processing by using the respective expanded data. For example, as an example of processing performed by the display process 180a, processing illustrated in FIG. 15 can be included. In the CPU 150, as long as a processing unit corresponding to processing to be performed is virtually implemented, all of the processing units described in the above first embodiment are not necessarily required to operate.

The display program 170a described above is not necessarily required to be stored in the HDD 170 or the ROM 160 from the beginning. For example, the display program 170a is stored in a "portable physical medium" such as a flexible disk inserted into the computer 100, a so-called FD, a CD-ROM, a DVD disk, an magneto-optical disk, and an IC card. The display program 170a may be acquired from these portable physical medium by the computer 100, to be executed. Moreover, the display program 170a is stored in another computer or server device that is connected to the computer 100 through a public line, the Internet, a LAN, a wide aera network (WAN), or the like. The display program 170a thus stored may be downloaded to the computer 100, to be executed.

## Claims

1. A display method comprising:
accepting(S101,S103) designation of a first workflow;
first counting(S106), by applying a plurality of objects to the first workflow, the number of object distributed by an element of a conditional branch of the first workflow for each of element at an end out of elements included in the first workflow;
second counting(S106), by applying the plurality of objects to a second workflow that is different from the first workflow, the number of objects distributed by an element of a conditional branch of the second workflow for each of element at an end out of elements included in the second workflow; and
displaying(S110) the number of objects distributed to an element at the end, associating with the element at the end, for each of the first workflow and the second workflow.

2. The display method according to claim 1, further including:
first calculating(S107), for each object included in the objects, a distance between a first symbol string corresponding to a first route on which the object flows from a start to an end of the first workflow and a second symbol string corresponding to a second route on which the object flows from a start to an end of the second workflow; and
second calculating(S108) a degree of difference of the first workflow and the second workflow based on the distance calculated for each of the objects, wherein the displaying includes displaying(S109,S110) a second workflow, the degree of difference of which satisfies a predetermined condition.

3. The display method according to claim 2, wherein the displaying includes displaying(S109,S110) the number of objects distributed to the element at the end associating with the element at the end, for each of the first workflow and a second workflow, the degree of difference of which is smallest.

4. The display method according to claim 2 wherein the distance is Levenshtein distance.

5. The display method according to claim 1, wherein the displaying includes changing(S110) a display form of an element of an edge connecting between elements of node according to the number of object distributed to the element of the edge.

6. The display method according to claim 1, wherein the displaying includes displaying(S110) an element at the end in which the number of object exceeds an upper limit in a display form different from a display form of other elements at the end.

7. A display program that causes a computer to execute a process comprising:
accepting(S101,S103) designation of a first workflow;
first counting(S106), by applying a plurality of objects to the first workflow, the number of object distributed by an element of a conditional branch of the first workflow for each of element at an end out of elements included in the first workflow;
second counting(S106), by applying the plurality of objects to a second workflow that is different from the first workflow, the number of objects distributed by an element of a conditional branch of the second workflow for each of element at an end out of elements included in the second workflow; and
displaying(S110) the number of objects distributed to an element at the end, associating with the element at the end, for each of the first workflow and the second workflow.

8. The display program according to claim 7, the process further including:
first calculating(S107), for each object included in the objects, a distance between a first symbol string corresponding to a first route on which the object flows from a start to an end of the first workflow and a second symbol string corresponding to a second route on which the object flows from a start to an end of the second workflow; and
second calculating(S108) a degree of difference of the first workflow and the second workflow based on the distance calculated for each of the objects, wherein the displaying includes displaying(S109,S110) a second workflow, the degree of difference of which satisfies a predetermined condition.

9. The display program according to claim 8, wherein the displaying includes displaying(S109,S110) the number of objects distributed to the element at the end associating with the element at the end, for each of the first workflow and a second workflow, the degree of difference of which is smallest.

10. The display program according to claim 8 wherein the distance is Levenshtein distance.

11. The display program according to claim 7, wherein the displaying includes changing(S110) a display form of an element of an edge connecting between elements of node according to the number of object distributed to the element of the edge.

12. The display program according to claim 7, wherein the displaying includes displaying(S110) an element at the end in which the number of object exceeds an upper limit in a display form different from a display form of other elements at the end.

13. An information processing apparatus(10) comprising:
an accepting unit(16) configured to accept designation of a first workflow;
a first counting unit(17A) configured to count, by applying a plurality of objects to the first workflow, the number of object distributed by an element of a conditional branch of the first workflow for each of element at an end out of elements included in the first workflow;
a second counting unit(17A) configured to count, by applying the plurality of objects to a second workflow that is different from the first workflow, the number of objects distributed by an element of a conditional branch of the second workflow for each of element at an end out of elements included in the second workflow; and
a display unit(18) configured to display the number of objects distributed to an element at the end, associating with the element at the end, for each of the first workflow and the second workflow.

14. The information processing apparatus according to claim 13, further including:
a first calculating unit(17B) configured to calculate, for each object included in the objects, a distance between a first symbol string corresponding to a first route on which the object flows from a start to an end of the first workflow and a second symbol string corresponding to a second route on which the object flows from a start to an end of the second workflow; and
a second calculating unit(17B) configured to calculate a degree of difference of the first workflow and the second workflow based on the distance calculated for each of the objects, wherein the displaying unit(18) displays a second workflow, the degree of difference of which satisfies a predetermined condition.

15. The information processing apparatus according to claim 14, wherein the displaying unit(18) displays the number of objects distributed to the element at the end associating with the element at the end, for each of the first workflow and a second workflow, the degree of difference of which is smallest.

16. The information processing apparatus according to claim 14, wherein the distance is Levenshtein distance.

17. The information processing apparatus according to claim 13, wherein the displaying unit(18) changes a display form of an element of an edge connecting between elements of node according to the number of object distributed to the element of the edge.

18. The information processing apparatus according to claim 13, wherein the displaying unit(18) displays an element at the end in which the number of object exceeds an upper limit in a display form different from a displays form of other elements at the end.
